# EUROPÄISCHE PATENTSCHRIFT

(11) **EP 3 229 920 B1**
(45) Veröffentlichungstag und Bekanntmachung des Hinweises auf die Patenterteilung: **04.12.2019**
(21) Anmeldenummer: 15805475.9
(22) Anmeldetag: 07.12.2015
(51) Int. Cl.: A61Q 5/12, A61Q 5/02, A61Q 5/00, A61K 8/65, A61K 8/64, A61K 8/46, A61K 8/58

(54) **HAARBEHANDLUNGSMITTEL MIT METHIONYLMETHIONIN UND POLYORGANOSILOXAN**
HAIR TREATMENT COMPOSTITION WITH METHIONYLMETHIONINE AND A POLYORGANOSILOXAN
TRAITEMENT DES CHEVEUX AVEC METHIONYLMETHIONINE ET UNE POLYORGANOSILOXAN

(30) Priorität: 09.12.2014 DE 102014225209
(43) Veröffentlichungstag der Anmeldung: 18.10.2017
(73) Patentinhaber: Evonik Degussa GmbH, 45128 Essen (DE)
(72) Erfinder: SCHULZE ZUR WIESCHE, Erik, 22453 Hamburg (DE); SCHEUNEMANN, Volker, 21339 Lüneburg (DE)
(74) Vertreter: Evonik Patent Association
(86) Internationale Anmeldenummer: PCT/EP2015/078776
(87) Internationale Veröffentlichungsnummer: WO 2016/091781

(56) Entgegenhaltungen:
- WO-A1-00/51556
- WO-A1-2007/068401
- WO-A2-2013/079299

## Beschreibung

Die Erfindung betrifft kosmetische Zusammensetzungen, insbesondere Haarbehandlungsmittel wie Shampoos und sogenannte Konditionierer, mit einer Wirkstoffkombination zur schonenden und effektiven Pflege der Haare.

Nicht zuletzt durch starke Beanspruchung der Haare, beispielsweise durch Färben oder Dauerwellen als auch durch Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Pflegebehandlungen beispielsweise die Nass- und Trockenkämmbarkeit des Haares, der Halt und die Fülle des Haares optimiert sein oder die Haare vor erhöhtem Spliss geschützt sein.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splissrate verringert. Siehe z.B. WO00/51556 A, WO2007/068401 A1.

Ebenso sind im Stand der Technik multifunktionale kosmetische Produkte bekannt. Insbesondere zählen hierzu die so genannten "2 in 1" Shampoos, welche nicht nur das Haar reinigen, sondern es auch konditionieren. Derartige Produkte werden vom Verbraucher sehr geschätzt, weil sie durch ihre Produktleistung mindestens einen Verfahrensschritt, beispielsweise das Konditionieren mit einer klassischen Haarspülung, erübrigen

Silikone und insbesondere aminofunktionelle Silikone sind als Pflegestoffe in Haarbehandlungsmitteln bekannt, und entsprechende Produkte sind im Markt weit verbreitet. Aus der WO 2013/079299 A2 sind kosmetische Zusammensetzungen mit aminofunktionellen Polyorganosiloxanen bekannt, welche zahlreiche Eigenschaften der mit ihnen behandelten Körperoberflächen, insbesondere Haare, verbessern und beispielsweise neben verbesserten Kämmbarkeiten und verbessertem Griff führen. Es besteht aber weiterhin der Bedarf, die erzielten Effekte, insbesondere im Hinblick auf den Griff, die Kämmbarkeit, die Weichheit und das Volumen der Haare und der Frisur zu verbessern und kosmetische Mittel diesbezüglich weiterzuentwickeln.

Es wurde nun gefunden, dass eine Kombination von bestimmten Polyorganosiloxanen mit Methionylmethionin eine besonders positive Wirkung auf Körperoberflächen, insbesondere auf die Haut, das Haar und den Haarfollikel besitzt

Ein erster Gegenstand der vorliegenden Erfindung sind kosmetische Mittel, enthaltend -jeweils bezogen auf ihr Gewicht -
a) 0,0001 bis 20 Gew.-% Polyorganosiloxan der Formel (I) 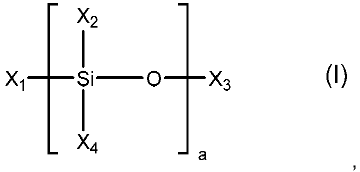 worin
   X₁ und X₂ unabhängig voneinander OH, OR¹, R², O-PDMS oder O-fSiloxan bedeuten,
   X₃ Wasserstoff oder einen einwertigen Kohlenwasserstoffrest mit 1 bis 8 Kohlenstoffatomen je Rest, PDMS oder fSiloxan bedeutet,
   X₄ ein Rest der Formel
   -CH₂NHR⁴, -CH₂NR⁴₂ oder 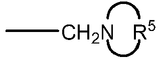 ist und
   a eine Zahl von 1 bis 100 ist,
   wobei
   R¹ einen Alkylrest mit 1 bis 8 Kohlenstoffatomen bedeutet,
   R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
   PDMS für 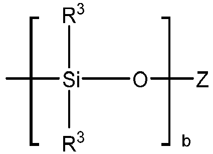 steht,
   fSiloxan für 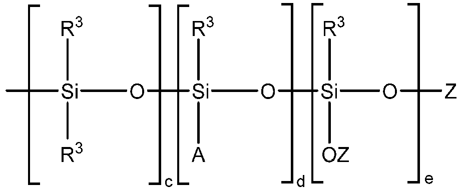 steht,
   R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
   A ein Rest der Formel R⁶-[NR⁷-R⁸-]_{f}NR⁷₂ bedeutet,
   wobei
   R⁶ ein zweiwertiger linearer oder verzweigter Kohlenwasserstoffrest mit 3 bis 18 Kohlenstoffatomen bedeutet,
   R⁷ein Wasserstoffatom, einen Alkylrest mit 1 bis 8 Kohlenstoffatomen oder einen Acylrest bedeutet, R⁸ ein zweiwertiger Kohlenwasserstoffrest mit 1 bis 6 Kohlenstoffatomen bedeutet,
   b eine Zahl von 1 bis 2000 ist,
   c 0 oder eine Zahl von 1 bis 2000 ist,
   d eine Zahl von 1 bis 1000 ist,
   e 0 oder eine Zahl von 1 bis 5 ist,
   f 0, 1, 2, 3 oder 4 ist,
   Z Wasserstoff, einen Alkylrest mit 1 bis 8 Kohlenstoffatomen oder 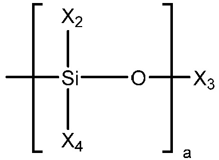 bedeutet,
   R⁴ einen einwertigen, gegebenenfalls N- und/oder O-Atome enthaltenden Kohlenwasserstoffrest mit 1 bis 18 Kohlenstoffatomen bedeutet und
   R⁵ einen zweiwertigen gegebenenfalls N- und/oder O-Atome enthaltenden Kohlenwasserstoffrest mit 3 bis 12 Kohlenstoffatomen bedeutet. und
b) 0,0001 bis 30 Gew.-% Methionylmethionin.

Das Mittel ist ein kosmetisches Mittel. Bevorzugte kosmetische Mittel sind ausgewählt aus der Gruppe der Duschgele, Duschbäder, Zahnreinigungsmittel, Mundwässer, Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen.

Besonders bevorzugte kosmetische Mittel dienen der Behandlung keratinischer Fasern und stellen somit Haarbehandlungsmittel dar. Haarbehandlungsmittel im Sinne der vorliegenden Erfindung sind beispielsweise Haarshampoos, Haarkonditionierer, konditionierende Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Im Hinblick auf die Tatsache, dass insbesondere Männer oft die Anwendung mehrerer unterschiedlicher Mittel und/oder mehrere Anwendungsschritte scheuen, sind solche Mittel bevorzugt, die der Mann ohnehin anwendet. Bevorzugte Mittel sind daher Shampoos, Haarkonditionierer oder Haar-Tonics.

Die Mittel enthalten - bezogen auf ihr Gewicht - 0,0001 bis 20 Gew.-% mindestens eines ausgewählten Polyorganosiloxans.

Die Mittel enthalten als ersten wesentlichen Inhaltsstoff mindestens ein Polyorganosiloxan der Formel (I) 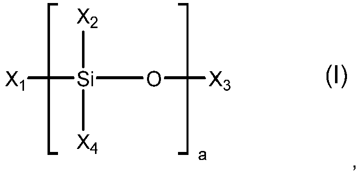 worin
X₁ und X₂ unabhängig voneinander OH, OR¹, R², O-PDMS oder O-fSiloxan bedeuten,
X₃ Wasserstoff oder einen einwertigen Kohlenwasserstoffrest mit 1 bis 8 Kohlenstoffatomen je Rest, PDMS oder fSiloxan bedeutet,
X₄ ein Rest der Formel
-CH₂NHR⁴, CH₂NR⁴₂ oder 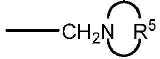 ist und
a eine Zahl von 1 bis 100, vorzugsweise eine Zahl von 1 bis 5, ist,
wobei
R¹ einen Alkylrest mit 1 bis 8 Kohlenstoffatomen bedeutet,
R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet, PDMS für 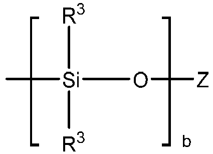 steht,
fSiloxan für 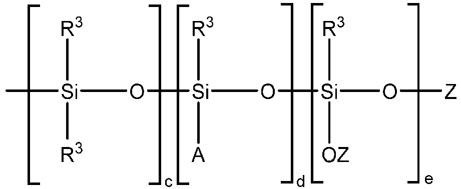 steht,
R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
A ein Rest der Formel R⁶-[NR⁷-R⁸-]_{f}NR⁷₂ bedeutet,
wobei
R⁶ ein zweiwertiger linearer oder verzweigter Kohlenwasserstoffrest mit 3 bis 18 Kohlenstoffatomen bedeutet,
R⁷ ein Wasserstoffatom, einen Alkylrest mit 1 bis 8 Kohlenstoffatomen oder einen Acylrest bedeutet, R⁸ ein zweiwertiger Kohlenwasserstoffrest mit 1 bis 6 Kohlenstoffatomen bedeutet,
b eine Zahl von 1 bis 2000, vorzugsweise von 1 bis 1000, ist,
c 0 oder eine Zahl von 1 bis 2000, vorzugsweise von 50 bis 1000, ist,
d eine Zahl von 1 bis 1000, vorzugsweise von 1 bis 10, ist,
e 0 oder eine Zahl von 1 bis 5 ist,
f 0, 1, 2, 3 oder 4 ist,
Z Wasserstoff einen Alkylrest mit 1 bis 8 Kohlenstoffatomen oder 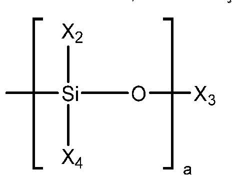 bedeutet,
R⁴ einen einwertigen, gegebenenfalls N- und/oder O-Atome enthaltenden Kohlenwasserstoffrest mit 1 bis 18 Kohlenstoffatomen bedeutet und
R⁵ einen zweiwertigen gegebenenfalls N- und/oder O-Atome enthaltenden Kohlenwasserstoffrest mit 3 bis 12 Kohlenstoffatomen bedeutet.

Beispiele für einen Alkylrest R¹ sind Methyl-, Ethyl-, *n*-Propyl-, *iso*-Propyl-, 1-*n*-Butyl-, 2-*n*-Butyl-, *iso-*Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso*-Octyl oder 2,2,4-Trimethylpentyl-, wobei Methyl-, Ethyl- und Butyl- bevorzugt sind.

Beispiele für Kohlenwasserstoffreste R² und R³ sind Alkylreste, wie Methyl-, Ethyl-, *n*-Propyl-, *iso*-Propyl-, 1-*n*-Butyl-, 2-*n*-Butyl-, *iso*-Butyl-, *tert*.-Butyl-, *n*-Pentyl-, *iso*-Pentyl-, *neo*-Pentyl-, *tert*.-Pentyl, *n*-Hexyl, *n-*Heptyl, *n*-Octyl, *iso*-Octyl, 2,2,4-Trimethylpentyl-, *n*-Nonyl-, *n*-Decyl-, *n*-Dodecyl-, *n*-Octadecyl-, Cyclopentyl-, Cyclohexyl-, Cycloheptyl-, Methylcyclohexyl-, Vinyl-, 5-Hexenyl-, Cyclohexenyl-, 1-Propenyl-, Allyl-, 3-Butenyl-, 4-Pentenyl-, Phenyl-, Naphthyl-, Anthryl-, Phenanthryl-, o-Tolyl-, m-Tolyl, p-Tolyl-, Xylyl-, Ethylphenyl-, Benzyl-, alpha-Phenylethyl- und beta-Phenylethylreste. Bevorzugt sind als Rest R² der Methyl-, Ethyl-, Octyl- und Phenylrest, besonders bevorzugt sind der Methyl- und Ethylrest.

Beispiele für halogenierte Reste R² und R³ sind der 3,3,3-Trifluor-*n*-propyl-, 2,2,2,2',2',2'-Hexafluorisopropyl-, Heptafluorisopropyl-, o-Chlorphenyl-, m-Chlorphenyl und p-Chlorphenylrest.

Beispiele für R⁴ sind die für die Kohlenwasserstoffreste R² und R³ aufgeführten Alkyl-, Cycloalkyl-, Aryl-, Alkaryl- und Aralkylreste.

Bevorzugte Beispiele für R⁵ sind Reste der Formeln -CH₂-CH₂-O-CH₂-CH₂-, -CH₂-CH₂-NH-CH₂-CH₂- oder -CH₂-CH₂-NH-CH₂-, wobei der Rest -CH₂-CH₂-O-CH₂-CH₂- besonders bevorzugt ist.

Beispiele für R⁶ sind Alkylenrest mit 3 bis 10 Kohlenstofftomen wie Propylen, Butylen, Pentylen, Hexylen, Heptylen, Octylen, Nonylen und Decylen.

R⁷ kann ein Wasserstoffatom, ein Methyl-, Ethyl-, *n*-Propyl-, *iso*-Propyl-, 1-*n*-Butyl-, 2-*n*-Butyl-, *iso*-Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso*-Octyl, 2,2,4-Trimethylpentyl- oder Acetylrest sein, wobei ein Wasserstoffatom bevorzugt ist.

Bevorzugte Beispiele für R⁸ sind Alkylenreste wie Methylen, Ethylen, Propylen, Butylen, Pentylen, oder Hexylen.

Z ist bevorzugt Wasserstoff oder Methyl-, Ethyl-, *n*-Propyl-, *iso*-Propyl-, 1-*n*-Butyl-, 2-*n*-Butyl-, *iso*-Butyl-, *tert*.-Butyl-, *n*-Pentyl-, *iso*-Pentyl-, *neo*-Pentyl-, *tert*.-Pentyl, *n*-Hexyl, *n*-Heptyl, *n*-Octyl, *iso*-Octyl oder 2,2,4-Trimethylpentyl-, wobei Wasserstoff, Methyl-, Ethyl- und Butyl- besonders bevorzugt sind.

Bevorzugte Reste X₄ sind entsprechend der obigen Definitionen für R⁴ und R⁵ Aminomethyl-, Methylaminomethyl-, Dimethylaminomethyl-, Diethylaminomethyl-, Dibutylainomethyl-, Cyclohexylaminomethyl-, Morpholinomethyl-, Piperidinomethyl-, Piperazinomethyl-, ((Diethoxymethylsilyl)methyl)cyclohexylaminomethyl-, ((Triethoxysilyl)methyl)cyclohexylaminomethyl-, Anilinomethyl-, 3-Dimethylaminopropyl-aminomethyl-, Bis(3-dimethylaminopropyl)aminomethylrest und Mischungen daraus. Dabei ist es höchst bevorzugt, wenn das Haarbehandlungsmittel Polyorganosiloxane der Formel (I) enthält, die als Rest X₄ den Morpholinomethylrest enthalten.

Entsprechend der Definitionen für R⁶, R⁷ und R⁸ sind bevorzugte Beispiele für Rest A:
-(CH₂)₃NH₂
-(CH₂)₃-NH-(CH₂)₂-NH₂
-CH₂CH(CH₃)CH₂-NH-(CH₂)₂-NH₂
-(CH₂)₃-NH(Cyclohexyl)
-(CH₂)₃-NHCH₃
-(CH₂)₃-N(CH₃)₂
-(CH₂)₃-NHCH₂CH₃
-(CH₂)₃-N(CH₂CH₃)₂
-(CH₂)₄-NH₂
-CH₂CH(CH₃)CH₂-NH₂
-(CH₂)₃-NH-(CH₂)₂-NHCH₃
-(CH₂)₃-NH-(CH₂)₂-N(CH₃)₂
-(CH₂)₃-NH-(CH₂)₂-NHCH₂CH₃
-(CH₂)₃-NH-(CH₂)₂-N(CH₂CH₃)₂
-(CH₂)₃[-NH-CH₂CH₂]₂-NH₂
-(CH₂)₃-NH(Acetyl)
-(CH₂)₃-NH-(CH₂)₂-NH(Acetyl) und
-(CH₂)₃-N(Acetyl)-(CH₂)₂-NH(Acetyl).

Zur Herstellung der Polyorganosiloxane der Formel (I) werden vorzugsweise handelsübliche Polydimethylsiloxane mit endständigen Silanolgruppen und/oder Polydimethylsiloxane mit endständigen Alkoxy- und Silanolgruppen und/oder Amin-funktionalisierte Siloxane, die Silanolgruppen oder Alkoxy- und Silanolgruppen enthalten, mit einem Dialkoxy- und/oder Trialkoxysilan, welches einen Rest Formel
-CH₂NHR⁴, -CH₂NR⁴₂ oder 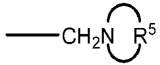 aufweist, umgesetzt.

Entsprechend steht in Formel (I) "fSiloxan" für einen Rest, der sich von einem Amin-funktionalisierten Siloxan ableitet.

Besonders bevorzugt werden Trialkoxysilane oder eine Mischung von Dialkoxy- und Trialkoxysilanen, eingesetzt, wobei der Einsatz von Trialkoxysilanen alleine besonders bevorzugt ist. Beim Einsatz von Trialkoxysilanen oder einer Mischung von Dialkoxy- und Trialkoxysilanen werden unabhängig von der Struktur der eingesetzten Siloxane und der Position der Alkoxy- und/oder Silanolgruppen in den Siloxanen zumindest teilweise vernetzte Polyorganopolysiloxane erhalten. In einer ganz besonders bevorzugten Ausführungsform enthält das kosmetische Mittel vernetzte Polyorganosiloxane. In einer höchst bevorzugten Ausführungsform enthält das kosmetische Mittel vernetzte Polyorganosiloxane, die aus der Umsetzung von Siloxanen und Trialkoxysilanen hervorgegangen sind.

Bevorzugte Beispiele für die eingesetzten Dialkoxy- oder Trialkoxysilane umfassen:
Diethylaminomethylmethyldimethoxysilan,
Dibutylaminomethyltriethoxysilan,
Dibutylaminomethyltributoxysilan,
Cyclohexylaminomethyltrimethoxysilan,
Cyclohexylaminomethyltriethoxysilan,
Cyclohexylaminomethyl-methyldiethoxysilan,
Anilinomethyltriethoxysilan,
Anilinomethylmethyldiethoxysilan,
Morpholinomethyltriethoxysilan,
Morpholinomethyltrimethoxysilan,
Morpholinomethyltriisopropoxysilan,
3-Dimethylaminopropyl-aminomethyltrimethoxysilan,
Morpholinomethyltributoxysilan,
Morpholinomethyltrialkoxysilan, wobei der Alkoxyrest ein C₁-C₄-Alkoxyrest ist, insbesondere ein Gemisch aus Methoxy- und Ethoxyrest ist,
Piperazinomethyltriethoxysilan,
Piperidinomethyltriethoxysilan und
Teilhydrolysate davon.

Ein besonders bevorzugtes Silan ist Morpholinomethyltriethoxysilan.

Ein besonders bevorzugt einsetzbares Amin-funktionalisiertes Siloxan ist ein Copolymer aus 3-(2-Aminoethylamino)propylmethylsiloxy- und Dimethylsiloxyeinheiten, welches Silanolgruppen oder Alkoxy- und Silanolgruppen aufweist.

Bei der Herstellung der Polyorganosiloxane der Formel (I) können gleiche oder verschiedene Siloxane sowie gleiche oder verschiedene Silane eingesetzt werden.

Ein insbesondere bevorzugtes Polyorganosiloxan ist beispielsweise unter der INCI-Bezeichnung Amodimethicone/Morpholinomethyl Silsesquioxane Copolymer und/oder unter der CAS-Nummer 1293390-78-9 bekannt. Kommerziell erhältlich ist dieses Polyorganosiloxan unter der Bezeichnung Belsil® ADM 8301 E (ex Wacker). Der Rohstoff stellt eine Mikroemulsion dar und weist die folgenden Bestandteile auf: Amodimethicone/Morpholinomethyl Silsesquioxane Copolymer, Trideceth-5, Glycerin, Phenoxyethanol und Wasser.

Die Polyorganosiloxane können je nach Anwendungszweck der kosmetischen Mittel in variierenden Mengen eingesetzt werden. Die kosmetischen Mittel enthalten das Polyorganoslioxan bevorzugt in einer Menge von 0,01 bis 10,0 Gew.-% und besonders bevorzugt von 0,05 bis 2,0 Gew.-%, jeweils bezogen auf das Gewicht des anwendungsbereiten Mittels.

Der Einsatz der Polyorganosiloxane erfolgt vorzugsweise als wässrige Suspensionen oder wässrige Emulsionen von Polyorganosiloxanen. Die Dispersionen können ein oder mehrere Tenside als Dispergatoren enthalten. Die Tenside können von beliebiger Art sein, ionisch und/oder nichtionisch. Alternativ können anorganische Feststoffe wie Kieselsäuren und/oder Bentonite als Dispergatoren eingesetzt sein. Die mittels Lichtstreuung in den Dispersionen gemessene mittlere Teilchengrösse der Polyorganosiloxane liegt vorzugsweise im Bereich 0,001 bis 100 µm, mehr bevorzugt bei 0,002 bis 10 µm. Die pH-Werte können von 1 bis 14 variieren. Bevorzugt liegt der pH-Wert von 3 bis 9, besonders bevorzugt von 5 bis 8.

Die Mittel enthalten als zweiten wesentlichen Inhaltstoff- bezogen auf ihr Gewicht - 0,0001 bis 30 Gew.-% Methionylmethionin.

Methionylmethionin ist das Dipeptid von Methionin und weist folgende Struktur auf:

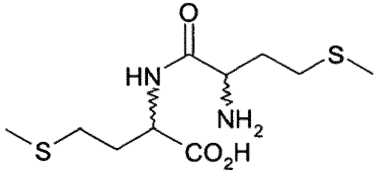

Vom Dipeptid Methionylmethionin existieren die vier verschiedenen Stereoisomere DD, LL, DL und LD. Von den vier Stereoisomeren ist nur das L-Methionyl-L-methionin natürlich, die drei anderen Dipeptide L-Methionyl-D-methionin, D-Methionyl-L-methionin und D-Methionyl-D-methionin sind alle nicht natürlich. Die beschriebenen positiven Wirkungen können mit allen vier stereoisomeren Dipeptiden erzielt werden. Das Methionylmethionin kann als DD/LL/LD/DL-Mischung, als DL/LD-Mischung oder DD/LL-Mischung eingesetzt werden. Prinzipiell ist auch der Einsatz eines stereoisomeren Dipeptids alleine oder in Kombination mit einem oder zwei weiteren Stereoisomeren denkbar. Wird nur ein stereoisomeres Dipeptid eingesetzt, werden vorzugsweise das L-Methionyl-L-methionin oder das D-Methionyl-D-methionin gewählt.

Die beiden Paare DD/LL-Methionylmethionin und DL/LD-Methionylmethionin sind diastereomer zueinander und haben unterschiedliche physikalische Daten. So besitzt beispielsweise das Diastereomerenpaar DD/LL bei Raumtemperatur eine Löslichkeit von 21,0 g/l in Wasser, wohingegen die Löslichkeit vom Diastereomerenpaar DL/LD bei 0,4 g/l liegt. Entsprechend kann es bevorzugt sein, das Methionylmethionin als DD/LL-Mischung oder als DD/LL/LD/DL-Mischung mit einem hohen Anteil an dem DD/LL-Diastereomerenpaar einzusetzen. Diese Mischungen lassen sich einfach in einer wässrigen Phase oder einer wässrig-tensidischen Phase eines kosmetischen Mittels einarbeiten/lösen.

Die kosmetischen Mittel enthalten das Methionylmethionin bevorzugt in einer Menge von 0,01 bis 5,0 Gew.-% und besonders bevorzugt von 0,1 bis 1,0 Gew.-%, jeweils bezogen auf das Gewicht des anwendungsbereiten kosmetischen Mittels.

Es hat sich gezeigt, dass der Einsatz der vorstehend genannten Polyorganosiloxane in Kombination mit Methionylmethionin den kosmetischen Mitteln herausragende Eigenschaften verleiht. Die kosmetischen Mittel in Form von Haarbehandlungsmittel verleihen den mit ihnen behandelten Haaren mehr Spannkraft, was sich in höheren Zugfestigkeiten der Keratinfasern und in einer Verringerung der Elastizitätsabnahme, zum Beispiel bei Beschädigung durch atmosphärische Einwirkungen zeigt.

Insbesondere die besonders bevorzugten Morpholinomethyl-enthaltenen Polyorganosiloxane stabilisieren darüber hinaus in Kombination mit dem Methionylmethionin den Feuchtigkeitshaushalt der keratinischen Fasern, so dass sich die Kämmbarkeit verbessert und der Alterungsprozess verzögert wird. Ein weiterer Vorteil der kosmetischen Mittel in Form von Haarbehandlungsmitteln liegt darin, dass die Formbarkeit und Restrukturierbarkeit von mit ihnen behandelten keratinischen Fasern verbessert wird. Ebenso erfolgt durch die Kombination aus Polyorganosiloxanen und Methionylmethionin eine Hydrophobierung der Haaroberfläche. Durch die Hydrophobierung wird der Kontaktwinkel von Wassertropfen, die auf die behandelten Haare kommen, deutlich erhöht, was ein Maß für die Produktleistung, insbesondere Pflegeleistung, ist.

Darüber hinaus führen die kosmetischen Mittel bei Applikation auf die Haut (beispielsweise durch ein Schaumbad oder Duschgel) zu einer verbesserten Geschmeidigkeit der Haut.

Die kosmetischen Mittel können weitere Wirk- und Hilfsstoffe beinhalten. Diese werden nachfolgend beschrieben.

Es hat sich gezeigt, dass die Wirkung der kosmetischen Mittel mit den ausgewählten Polyorganosiloxanen und Methionylmethionin noch gesteigert werden kann, wenn bestimmte nichtionische Komponenten ebenfalls in den Mitteln eingesetzt werden. Zudem haben diese nichtionischen Komponenten positive Effekte auf die Lagerstabilität der Mittel. Nichtionische Komponenten, die hier besonders geeignet sind, sind Ethoxylate von Decanol, Undecanol, Dodecanol, Tridecanol usw. Als besonders geeignet haben sich ethoxylierte Tridecanole erwiesen, die mit besonderem Vorzug in die Mittel inkorporiert werden. Besonders bevorzugte kosmetische Mittel enthalten - bezogen auf ihr Gewicht - 0,00001 bis 5 Gew.-%, vorzugsweise 0,0001 bis 3,5 Gew.-%, besonders bevorzugt 0,001 bis 2 Gew.-%, weiter bevorzugt 0,01 bis 1 Gew.-% und insbesondere 0,1 bis 0,5 Gew.-% verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen.

Bevorzugte kosmetische Mittel sind dadurch gekennzeichnet, dass sie - bezogen auf ihr Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthalten.

Als anionische Tenside eignen sich für die kosmetischen Mittel alle für die Verwendung am menschlichen Körper geeigneten anionischen oberflächenaktiven Stoffe. Diese sind gekennzeichnet durch eine wasserlöslich machende, anionische Gruppe wie zum Beispiel eine Carboxylat-, Sulfat-, Sulfonat- oder Phosphat-Gruppe und eine lipophile Alkylgruppe mit etwa 8 bis 30 C-Atomen. Zusätzlich können im Molekül Glycol- oder Polyglycolether-Gruppen, Ester-, Ether- und Amidgruppen sowie Hydroxylgruppen enthalten sein. Amphotere Tenside, welche auch als zwitterionische Tenside bezeichnet werden, werden solche oberflächenaktiven Verbindungen genannt, die im Molekül mindestens eine quartäre Ammoniumruppe und mindestens eine -COO⁽⁻⁾ - oder -SO₃⁽⁻⁾ -Gruppe tragen. Besonders geeignete amphotere/zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes amphoteres/zwitterionisches Tensid ist das unter der INCI-Bezeichnung Cocamidopropyl Betaine bekannte Fettsäureamidderivat. Unter amphoteren Tensiden werden auch solche oberflächenaktiven Verbindungen verstanden, die außer einer C₈- C₂₄ -Alkyl- oder -Acylgruppe mindestens eine freie Aminogruppe und mindestens eine - COOH- oder -SO₃H-Gruppe enthalten und zur Ausbildung innerer Salze befähigt sind. Beispiele für geeignete amphotere/zwitterionische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere/zwitterionische Tenside sind das N-Kokosalkylaminopropionat, das Kokosacylaminoethylaminopropionat und das C₁₂- C₁₈-Acylsarcosin.

Nichtionische Tenside enthalten als hydrophile Gruppe zum Beispiel eine Polyolgruppe, eine Polyalkylenglycolethergruppe oder eine Kombination aus Polyol- und Polyglycolethergruppe. Ganz besonders bevorzugt sind kosmetische Mittel, die zusätzlich Fettalkohol(e) und/oder Fettalkoholalkoxylat(e), vorzugsweise C₁₂₋₂₂-Fettalkohol(e) und/oder C₁₂₋₂₂-Fettalkoholethoxylat(e) mit 10 bis 30 EO-Einheiten, besonders bevorzugt C₁₆₋₁₈-Fettalkohol(e) und/oder C₁₆₋₁₈-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht des kosmetischen Mittels, enthalten.

Zusammenfassend sind kosmetische Mittel, insbesondere Haarbehandlungsmittel, bevorzugt, die - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

H₃C-(CH₂)ₙ-(OCH₂CH₂)ₖ-OSO₃⁻ M⁺

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, stehen.

Als weiteren optionalen Bestandteil können die kosmetischen Mittel 0,01 bis 10 Gew.-% mindestens ein Polymer aus der Gruppe der kationischen und/oder amphoteren Polymere enthalten.

Unter kationischen oder amphoteren Polymeren sind Polymere zu verstehen, welche in der Haupt- und/oder Seitenkette eine Gruppe aufweisen, welche "temporär" oder "permanent" kationisch sein kann. Als "permanent kationisch" werden solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Zusätzlich zu kationischen Polymeren oder an ihrer Stelle können die Haarbehandlungsmittel auch amphotere Polymere enthalten. Diese weisen zusätzlich zu der mindestens einen kationisch geladenen Gruppe mindestens eine negativ geladene Gruppe im Molekül auf und werden auch als zwitterionische Polymere bezeichnet.

Vorzugsweise wird das Polymer oder werden die Polymere innerhalb engerer Mengenbereiche eingesetzt. So sind kosmetische Mittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% amphotere(s) Polymer(e), enthalten.

Unabhängig davon, ob in den Mitteln amphotere Polymere enthalten sind oder nicht, sind weiter bevorzugte kosmetische Mittel dadurch gekennzeichnet, dass sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten.

Weitere geeignete Inhaltsstoffe umfassen nichtionische Polymere, anionische Polymere, Fettstoffe, Wachse, Proteinhydrolysate, Aminosäuren, Oligopetide, Vitamine, Provitamine, Vitaminvorstufen, Betaine, Biochinone, Purin(derivate), Taurin(derivate), Pflegestoffe, Pflanzenextrakte, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle, weitere Silikone und/oder Konservierungsmittel.

Die kosmetischen Mittel weisen vorteilhafte Eigenschaften auf und verleihen den mit ihnen behandelten Körperoberflächen und/oder Haaren ebenfalls vorteilhafte Eigenschaften. Insbesondere bei der Haar- und Kopfhautbehandlung wurden Vorteile beobachtet, die über die Vorteile der Polyorganosiloxane oder des Methionylmethionins alleine hinausgehen. So steigern die Haarbehandlungsmittel die Elastizität der mit ihnen behandelten Haare und führen zu einer innerstrukturellen Stärkung der Haarfasern, welche sich zum Beispiel in höheren Schmelztemperaturen bei der Differenzthermoanalyse niederschlägt. Ebenso kommt es zu einer Hydrophobierung der Haaroberfläche, die sich in einer Erhöhung des Kontaktwinkels von Wassertropfen auf der Haaroberfläche zeigt. Es zeigt sich auch eine Verbesserung der Nass- und Trockenkämmbarkeiten. Auf der Haut und insbesondere der Kopfhaut bewirken die kosmetischen Mittel eine Erhöhung der Elastizität und überraschenderweise sebumregulierende Effekte. Der optische Eindruck "fettiger" Haut oder Haare wird damit vermieden oder abgeschwächt.

Ein weiterer Gegenstand der vorliegenden Erfindung ist die Verwendung von Methionylmethionin in einem kosmetischen Mittel, vorzugsweise in einem Haarbehandlungsmittel, umfassend Polyorganosiloxan der Formel (I) 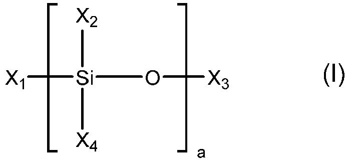 besitzt,
worin
X₁ und X₂ unabhängig voneinander OH, OR¹, R², O-PDMS oder O-fSiloxan bedeuten,
X₃ Wasserstoff oder einen einwertigen Kohlenwasserstoffrest mit 1 bis 8 Kohlenstoffatomen je Rest, PDMS oder fSiloxan bedeutet,
X₄ ein Rest der Formel
-CH₂NHR⁴, CH₂NR⁴₂ oder 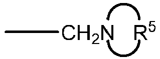 ist und
a eine Zahl von 1 bis 100 ist,
wobei
R¹ einen Alkylrest mit 1 bis 8 Kohlenstoffatomen bedeutet,
R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
PDMS für 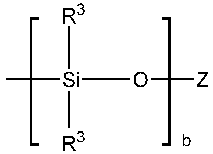 steht,
fSiloxan für 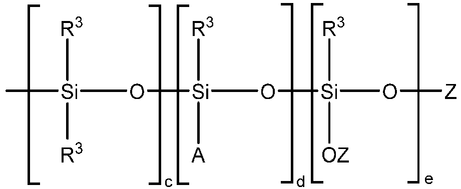 steht,
R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
A ein Rest der Formel R⁶-[NR⁷-R⁸-]_{f}NR⁷₂ bedeutet,
wobei
R⁶ ein zweiwertiger linearer oder verzweigter Kohlenwasserstoffrest mit 3 bis 18 Kohlenstoffatomen bedeutet,
R⁷ ein Wasserstoffatom, einen Alkylrest mit 1 bis 8 Kohlenstoffatomen oder einen Acylrest bedeutet, R⁸ ein zweiwertiger Kohlenwasserstoffrest mit 1 bis 6 Kohlenstoffatomen bedeutet,
b eine Zahl von 1 bis 2000 ist,
c 0 oder eine Zahl von 1 bis 2000 ist,
d eine Zahl von 1 bis 1000 ist,
e 0 oder eine Zahl von 1 bis 5 ist,
f 0, 1, 2, 3 oder 4 ist,
Z Wasserstoff, einen Alkylrest mit 1 bis 8 Kohlenstoffatomen oder 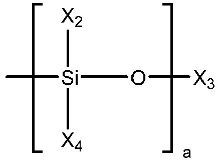 bedeutet,
R⁴ einen einwertigen, gegebenenfalls N- und/oder O-Atome enthaltenden Kohlenwasserstoffrest mit 1 bis 18 Kohlenstoffatomen bedeutet und
R⁵ einen zweiwertigen gegebenenfalls N- und/oder O-Atome enthaltenden Kohlenwasserstoffrest mit 3 bis 12 Kohlenstoffatomen bedeutet, zur Verbesserung der
- Zugfestigkeit von keratinischen Fasern, insbesondere von menschlichen Haaren.

Dazu wird das kosmetische Mittel auf die keratinischen Fasern und/oder die Haut, vorzugsweise die Kopfhaut, aufgetragen, dort 5 Sekunden bis 10 Minuten belassen und dann ausgespült oder das kosmetische Mittel wird auf die keratinischen Fasern und/oder die Haut, vorzugsweise die Kopfhaut, aufgetragen und verbleibt dort.

Bezüglich weiterer bevorzugter Ausführungsformen der Verwendung gilt mutatis mutandis das zu den kosmetischen Mitteln Gesagte.

Die folgenden Beispiele sollen den Gegenstand der vorliegenden Erfindung erläutern ohne ihn jedoch zu beschränken.

### Beispiele

Alle Mengenangaben sind, soweit nicht anders vermerkt, Gewichtsteile an Aktivstoff. Die folgenden Rezepturen wurden unter Anwendung bekannter Herstellungsverfahren bereitgestellt.

**Haarspülung:**

| | Gew.-% |
|---|---|
| Cetearyl Alcohol | 5,0 |
| Quaternium-87 | 0,75 |
| Glycol Distearate | 1,0 |
| Amodimethicone/Morpholinomethyl Silsesquioxane Copolymer, Trideceth-5, Glycerin | 0,3 |
| Shea Butter (INCI: Butyrospermum Parkii (Shea) Butter) | 1,5 |
| L-Methionyl-L-methionin | 1,0 |
| Milchsäure | 0,1 |
| Behenoyl PG -Trimoniumchloride | 1,5 |
| Behentrimonium Chloride | 0,5 |
| Polyquaternium-37 | 0,25 |
| Glycerin | 0,1 |
| Dimethicone | 1,0 |
| Wasser, Konservierung, Begleitstoffe und ggf. Parfümöle | ad 100 |

**Pflegespray:**

| | Gew.-% |
|---|---|
| Stearamidopropyldimethylamine | 0,3 |
| Glycerin | 2,0 |
| Polyquaternium-10 | 0,2 |
| Amodimethicone/Morpholinomethyl Silsesquioxane Copolymer | 0,02 |
| Panthenol | 0,2 |
| DL-Methionyl-DL-methionin | 0,8 |
| Ethanol | 15 |
| Nicotinsäureamid | 0,1 |
| Dicaprylyl Carbonate | 0,1 |
| Cetrimonium Chloride | 0,2 |
| Wasser, Konservierung, Begleitstoffe und ggf. Parfümöle | ad 100 |

**Haarshampoo:**

| | Gew.-% |
|---|---|
| Sodium Laureth Sulfate | 9,0 |
| Ammonium Lauryl Sulfate | 5,0 |
| Disodium Cocoamphodiacetate | 2,0 |
| Cocoamidopropyl Betaine | 6,0 |
| D-Methionyl-D-methionin | 0,5 |
| Panthenol | 0,2 |
| Amodimethicone/Morpholinomethyl Silsesquioxane Copolymer, Trideceth-5, Glycerin | 0,06 |
| Citronensäure | 0,4 |
| Guar Hydroxypropyltrimonium Chloride | 0,4 |
| Natriumchlorid | 1,3 |
| Wasser, Konservierung, Begleitstoffe und ggf. Parfümöle | ad 100 |

Es wurden die folgenden Handelsprodukte eingesetzt:

| Handelsprodukt | INCI | Lieferant/Hersteller |
|---|---|---|
| Lanette® ○ | Cetearyl Alcohol | BASF SE |
| Varisoft W575 PG | Quaternium-87 | Evonik |
| Cutina® GMS-V oder Cutina® AGS | Glycol Distearate | BASF SE |
| Polymer JR400 | Polyquaternium-10 | Dow |
| Dehyquart® A CA | Cetrimonium Chloride | BASF SE |
| Synthalen® CR | Polyquaternium-37 | 3V Sigma |
| Quartamin® BTC 131 | Behenoyl PG -Trimoniumchloride | Kao Chemicals |
| Genamin® KDMP | Behentrimonium Chloride | Clariant |
| Tego Amid S 18 | Stearamidopropyldimethylamine | Evonik |
| Ucare® Polymer JR 400 | Polyquaternium-10 | Dow |
| Cetiol CC | Dicaprylyl Carbonate | BASF SE |
| Texapon N70 NA | Sodium Laureth Sulfate | BASF SE |
| Empicol AL 70 | Ammonium Lauryl Sulfate | Huntsman |
| Rewoteric AM 2 | Disodium Cocoamphodiacetate | Evonik |
| Tego Betain F50 | Cocoamidopropyl Betaine | Evonik |
| N-Hance 3196 | Guar Hydroxypropyltrimonium Chloride | Ashland |
| Belsil® ADM 8301 E | Amodimethicone / Morpholinomethyl Silsesquioxane Copolymer, Trideceth-5, Glycerin | Wacker Silicones |

## Patentansprüche

1. Kosmetisches Mittel, enthaltend -jeweils bezogen auf sein Gewicht-
a) 0,0001 bis 20 Gew.-% Polyorganosiloxane der Formel (I) 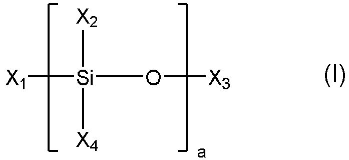 besitzt,
worin
X₁ und X₂ unabhängig voneinander OH, OR¹, R², O-PDMS oder O-fSiloxan bedeuten,
X₃ Wasserstoff oder einen einwertigen Kohlenwasserstoffrest mit 1 bis 8 Kohlenstoffatomen je Rest, PDMS oder fSiloxan bedeutet,
X₄ ein Rest der Formel
-CH₂NHR⁴, -CH₂NR⁴₂ oder 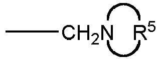 ist und
a eine Zahl von 1 bis 100 ist,
wobei
R¹ einen Alkylrest mit 1 bis 8 Kohlenstoffatomen bedeutet,
R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
PDMS für 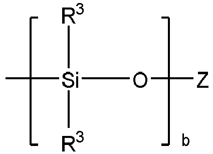 steht,
fSiloxan für 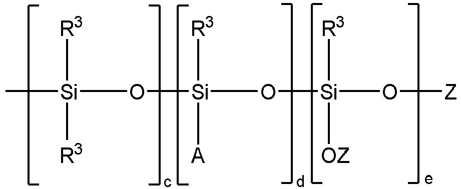 steht,
R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
A ein Rest der Formel R⁶-[NR⁷-R⁸-]_{f}NR⁷₂ bedeutet,
wobei
R⁶ ein zweiwertiger linearer oder verzweigter Kohlenwasserstoffrest mit 3 bis 18 Kohlenstoffatomen bedeutet,
R⁷ ein Wasserstoffatom, einen Alkylrest mit 1 bis 8 Kohlenstoffatomen oder einen Acylrest bedeutet,
R⁸ ein zweiwertiger Kohlenwasserstoffrest mit 1 bis 6 Kohlenstoffatomen bedeutet,
b eine Zahl von 1 bis 2000 ist,
c 0 oder eine Zahl von 1 bis 2000 ist,
d eine Zahl von 1 bis 1000 ist,
e 0 oder eine Zahl von 1 bis 5 ist,
f 0, 1, 2, 3 oder 4 ist,
Z Wasserstoff, einen Alkylrest mit 1 bis 8 Kohlenstoffatomen oder 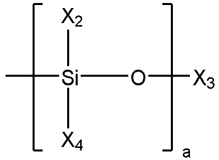 bedeutet,
R⁴ einen einwertigen, gegebenenfalls N- und/oder O-Atome enthaltenden Kohlenwasserstoffrest mit 1 bis 18 Kohlenstoffatomen bedeutet und
R⁵ einen zweiwertigen gegebenenfalls N- und/oder O-Atome enthaltenden Kohlenwasserstoffrest mit 3 bis 12 Kohlenstoffatomen bedeutet, und
b) 0,0001 bis 30 Gew.-% Methionylmethionin.

2. Kosmetisches Mittel nach Anspruch 1, **dadurch gekennzeichnet, dass** fSiloxan ein Amin-funktionalisiertes Siloxan, vorzugsweise ein Copolymer aus 3-(2-Aminoethylamino)propylmethylsiloxy- und Dimethylsiloxyeinheiten, ist.

3. Kosmetisches Mittel nach einem der Ansprüche 1 oder 2, **dadurch gekennzeichnet, dass** X₄ ausgewählt ist aus der Gruppe bestehend aus Aminomethyl-, Methylaminomethyl-, Dimethylaminomethyl-, Diethylaminomethyl-, Dibutylaminomethyl-, Cyclohexylaminomethyl-, Morpholinomethyl-, Piperidinomethyl-, Piperazinomethyl-, ((Diethoxymethylsilyl)methyl)cyclohexylaminomethyl-, ((Triethoxysilyl)methyl)cyclohexylaminomethyl-, Anilinomethyl-, 3-Dimethylaminopropyl-aminomethyl-, Bis(3-dimethylaminopropyl)aminomethyl- und Mischungen daraus.

4. Kosmetisches Mittel nach einem der Ansprüche 1 bis 3, **dadurch gekennzeichnet, dass** das Mittel Polyorganosiloxane enthält, die als Rest X₄ einen Morpholinomethylrest enthalten.

5. Kosmetisches Mittel nach einem der Ansprüche 1 bis 4, **dadurch gekennzeichnet, dass** das Mittel - bezogen auf sein Gewicht - weiterhin verzweigtes, ethoxyliertes Tridecanol (INCI-Bezeichnung: Trideceth-5) oder α-iso-Tridecyl-ω-hydroxypolyglycolether (INCI-Bezeichnung: Trideceth-10) oder deren Mischungen enthält.

6. Kosmetisches Mittel nach einem der Ansprüche 1 bis 5, **dadurch gekennzeichnet, dass** das Mittel das Methionylmethionin eine Mischung von L-Methionyl-L-methionin, L-Methionyl-D-methionin, D-Methionyl-L-methionin und D-Methionyl-D-methionin ist.

7. Haarbehandlungsmittel nach einem der Ansprüche 1 bis 5, **dadurch gekennzeichnet, dass** das Methionylmethionin L-Methionyl-L-methionin und/oder D-Methionyl-D-methionin ist.

8. Kosmetisches Mittel nach einem der Ansprüche 1 bis 7, enthaltend - jeweils bezogen auf sein Gewicht -
a) 0,0001 bis 20 Gew.-% eines Polyorganosiloxans, welches unter der INCI-Bezeichnung Amodimethicone/Morpholinomethyl Silsesquioxane Copolymer bekannt ist, und
b) 0,0001 bis 30 Gew.-% Methionylmethionin.

9. Kosmetisches Mittel nach einem der Ansprüche 1 bis 7, enthaltend - jeweils bezogen auf sein Gewicht -
a) 0,0001 bis 20 Gew.-% eines Polyorganosiloxans, welches durch Umsetzung eines Silanolgruppen oder Alkoxy- und Silanolgruppen enthaltenen Siloxans mit einem Trialkoxysilan oder einer Mischung aus Dialkoxy- und Trialkoxysilanen, wobei die Dialkoxy- und Trialkoxysilane einen Morpholinomethylrest aufweisen, erhältlich ist, und
b) 0,0001 bis 30 Gew.-% Methionylmethionin.

10. Verwendung von Methionylmethionin in einem kosmetischen Mittel, vorzugsweise in einem Haarbehandlungsmittel, umfassend Polyorganosiloxan der Formel (I) 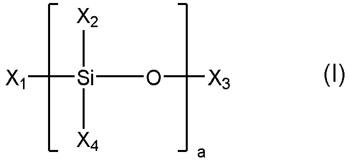 worin
X₁ und X₂ unabhängig voneinander OH, OR¹, R², O-PDMS oder O-fSiloxan bedeuten,
X₃ Wasserstoff oder einen einwertigen Kohlenwasserstoffrest mit 1 bis 8 Kohlenstoffatomen je Rest, PDMS oder fSiloxan bedeutet,
X₄ ein Rest der Formel
-CH₂NHR⁴, -CH₂NR⁴₂ oder 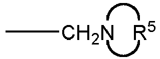 ist und
a eine Zahl von 1 bis 100 ist,
wobei
R¹ einen Alkylrest mit 1 bis 8 Kohlenstoffatomen bedeutet,
R² einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
PDMS für 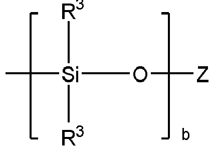 steht,
fSiloxan für 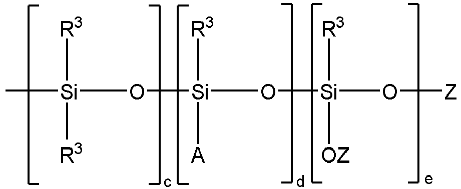 steht,
R³ unabhängig voneinander jeweils einen einwertigen, gegebenenfalls mit den Elementen N, P, S, O, Si und Halogen substituierten, gesättigten oder ungesättigten Kohlenwasserstoffrest mit 1 bis 200 Kohlenstoffatomen je Rest bedeutet,
A ein Rest der Formel R⁶-[NR⁷-R⁸-]_{f}NR⁷₂ bedeutet,
wobei
R⁶ ein zweiwertiger linearer oder verzweigter Kohlenwasserstoffrest mit 3 bis 18 Kohlenstoffatomen bedeutet,
R⁷ ein Wasserstoffatom, einen Alkylrest mit 1 bis 8 Kohlenstoffatomen oder einen Acylrest bedeutet, R⁸ ein zweiwertiger Kohlenwasserstoffrest mit 1 bis 6 Kohlenstoffatomen bedeutet,
b eine Zahl von 1 bis 2000 ist,
c 0 oder eine Zahl von 1 bis 2000 ist,
d eine Zahl von 1 bis 1000 ist,
e 0 oder eine Zahl von 1 bis 5 ist,
f 0, 1, 2, 3 oder 4 ist,
Z Wasserstoff, einen Alkylrest mit 1 bis 8 Kohlenstoffatomen oder 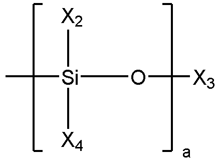 bedeutet,
R⁴ einen einwertigen, gegebenenfalls N- und/oder O-Atome enthaltenden Kohlenwasserstoffrest mit 1 bis 18 Kohlenstoffatomen bedeutet und
R⁵ einen zweiwertigen gegebenenfalls N- und/oder O-Atome enthaltenden Kohlenwasserstoffrest mit 3 bis 12 Kohlenstoffatomen bedeutet,
zur Verbesserung der Zugfestigkeit von keratinischen Fasern, insbesondere von menschlichen Haaren.

## Claims

1. Cosmetic composition comprising - in each case based on its weight - has
a) 0.0001 to 20% by weight polyorganosiloxanes of the formula (I) 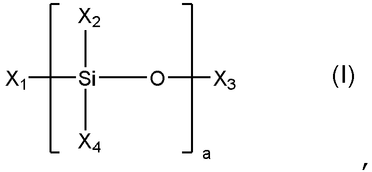 in which
X₁ and X₂ are mutually independently OH, OR¹, R², O-PDMS or O-fSiloxane,
X₃ is hydrogen or a monovalent hydrocarbon radical having 1 to 8 carbon atoms per radical, PDMS or fSiloxane,
X₄ is a radical of the formula
CH₂NHR⁴, CH₂NR⁴₂ or 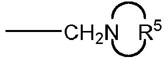 and
a is a number from 1 to 100,
where
R¹ is an alkyl radical having 1 to 8 carbon atoms, R² is a monovalent saturated or unsaturated hydrocarbon radical having 1 to 200 carbon atoms per radical, optionally substituted with the elements N, P, S, O, Si and halogen,
PDMS is 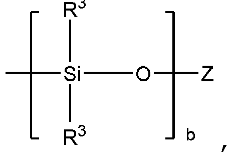 fSiloxane is 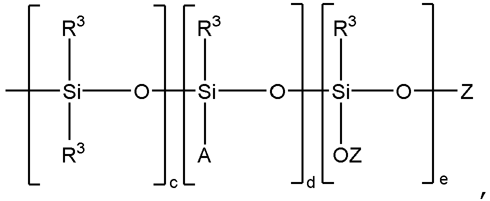 R³ is in each case mutually independently a monovalent saturated or unsaturated hydrocarbon radical having 1 to 200 carbon atoms per radical, optionally substituted with the elements N, P, S, O, Si and halogen,
A is a radical of the formula R⁶-[NR⁷-R⁸-]_{f}NR⁷2,
where
R⁶ is a divalent linear or branched hydrocarbon radical having 3 to 18 carbon atoms,
R⁷ is a hydrogen atom, an alkyl radical having 1 to 8 carbon atoms or an acyl radical,
R⁸ is a divalent hydrocarbon radical having 1 to 6 carbon atoms,
b is a number from 1 to 2000,
c is 0 or a number from 1 to 2000,
d is a number from 1 to 1000,
e is 0 or a number from 1 to 5,
f is 0, 1, 2, 3 or 4,
Z is hydrogen, an alkyl radical having 1 to 8 carbon atoms or 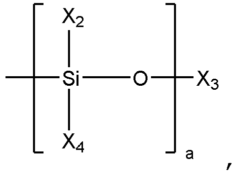 R⁴ is a monovalent hydrocarbon radical having 1 to 18 carbon atoms comprising optionally N and/or O atoms and
R⁵ is a divalent hydrocarbon radical having 3 to 12 carbon atoms comprising optionally N and/or O atoms, and
b) 0.0001 to 30% by weight methionylmethionine.

2. Cosmetic composition according to Claim 1, **characterized in that** fSiloxane is an amine-functionalized siloxane, preferably a copolymer of 3-(2-aminoethylamino)propylmethylsiloxy- and dimethylsiloxy units.

3. Cosmetic composition according to either of Claims 1 or 2, **characterized in that** X₄ is selected from the group consisting of aminomethyl-, methylaminomethyl-, dimethylaminomethyl-, diethylaminomethyl-, dibutylaminomethyl-, cyclohexylaminomethyl-, morpholinomethyl-, piperidinomethyl-, piperazinomethyl-, ((diethoxymethylsilyl)methyl)cyclohexylaminomethyl-, ((triethoxysilyl)-methyl)cyclohexylaminomethyl-, anilinomethyl-, 3-dimethylaminopropylaminomethyl-, bis(3-dimethylaminopropyl)aminomethyl- and mixtures thereof.

4. Cosmetic composition according to any of Claims 1 to 3, **characterized in that** the composition comprises polyorganosiloxanes, which comprise a morpholinomethyl radical as radical X₄.

5. Cosmetic composition according to any of Claims 1 to 4, **characterized in that** the composition - based on its weight - further comprises branched ethoxylated tridecanol (INCI name: Trideceth-5) or α-isotridecyl-ω-hydroxypolyglycol ether (INCI name: Trideceth-10) or mixtures thereof.

6. Cosmetic composition according to any of Claims 1 to 5, **characterized in that** the composition the methionylmethionine is a mixture of L-methionyl-L-methionine, L-methionyl-D-methionine, D-methionyl-L-methionine and D-methionyl-D-methionine.

7. Hair treatment composition according to any of Claims 1 to 5, **characterized in that** the methionylmethionine is L-methionyl-L-methionine and/or D-methionyl-D-methionine.

8. Cosmetic composition according to any of Claims 1 to 7, comprising - in each case based on its weight -
a) 0.0001 to 20% by weight of a polyorganosiloxane, which is known under the INCI name Amodimethicone/Morpholinomethyl Silsesquioxane Copolymer, and
b) 0.0001 to 30% by weight methionylmethionine.

9. Cosmetic composition according to any of Claims 1 to 7, comprising - in each case based on its weight -
a) 0.0001 to 20% by weight of a polyorganosiloxane, which is obtainable by reacting a siloxane, comprising silanol groups or alkoxy and silanol groups, with a trialkoxysilane or a mixture of dialkoxy- and trialkoxysilanes, wherein the dialkoxy- and trialkoxysilanes have a morpholinomethyl radical, and
b) 0.0001 to 30% by weight methionylmethionine.

10. Use of methionylmethionine in a cosmetic composition, preferably in a hair treatment composition, comprising polyorganosiloxanes of the formula (I) 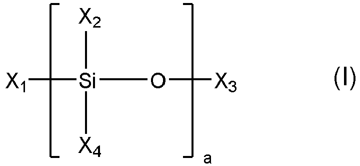 in which
X₁ and X₂ are mutually independently OH, OR¹, R², O-PDMS or O-fSiloxane,
X₃ is hydrogen or a monovalent hydrocarbon radical having 1 to 8 carbon atoms per radical, PDMS or fSiloxane,
X₄ is a radical of the formula
-CH₂NHR⁴, CH₂NR⁴₂ or 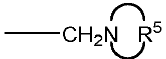 and
a is a number from 1 to 100,
where
R¹ is an alkyl radical having 1 to 8 carbon atoms,
R² is a monovalent saturated or unsaturated hydrocarbon radical having 1 to 200 carbon atoms per radical, optionally substituted with the elements N, P, S, O, Si and halogen,
PDMS is 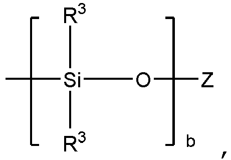 fSiloxane is 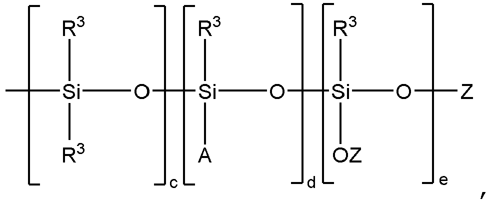 R³ is in each case mutually independently a monovalent saturated or unsaturated hydrocarbon radical having 1 to 200 carbon atoms per radical, optionally substituted with the elements N, P, S, O, Si and halogen,
A is a radical of the formula R⁶-[NR⁷-R⁸-]_{f}NR⁷₂,
where
R⁶ is a divalent linear or branched hydrocarbon radical having 3 to 18 carbon atoms,
R⁷ is a hydrogen atom, an alkyl radical having 1 to 8 carbon atoms or an acyl radical,
R⁸ is a divalent hydrocarbon radical having 1 to 6 carbon atoms,
b is a number from 1 to 2000,
c is 0 or a number from 1 to 2000,
d is a number from 1 to 1000,
e is 0 or a number from 1 to 5,
f is 0, 1, 2, 3 or 4,
Z is hydrogen, an alkyl radical having 1 to 8 carbon atoms or 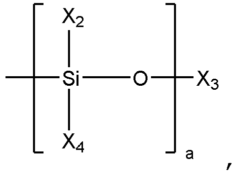 R⁴ is a monovalent hydrocarbon radical having 1 to 18 carbon atoms comprising optionally N and/or O atoms and R⁵ is a divalent hydrocarbon radical having 3 to 12 carbon atoms comprising optionally N and/or O atoms,
for improving the tensile strength of keratin fibres, particularly of human hair.

## Revendications

1. Agent cosmétique, contenant - à chaque fois par rapport à son poids - posséde
a) 0,0001 à 20% en poids de polyorganosiloxane de la formule (I) 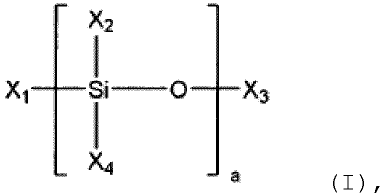 dans laquelle
X₁ et X₂ indépendamment l'un de l'autre signifient OH, OR¹, R², O-PDMS ou O-fsiloxane,
X₃ signifie hydrogène ou un radical hydrocarboné monovalent comportant 1 à 8 atome(s) de carbone par radical, PDMS ou fsiloxane,
X₄ est un radical de formule
-CH₂NHR⁴, -CH₂NR⁴₂ ou 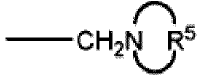 et a est un nombre de 1 à 100,
R¹ signifiant un radical alkyle comportant 1 à 8 atome(s) de carbone,
R² signifiant un radical hydrocarboné monovalent saturé ou insaturé, éventuellement substitué par les éléments N, P, S, O, Si et halogène, comportant 1 à 200 atome(s) de carbone par radical,
PDMS représentant 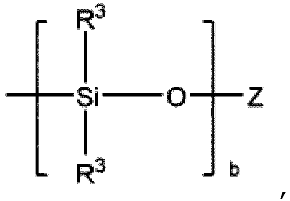 fsiloxane représentant 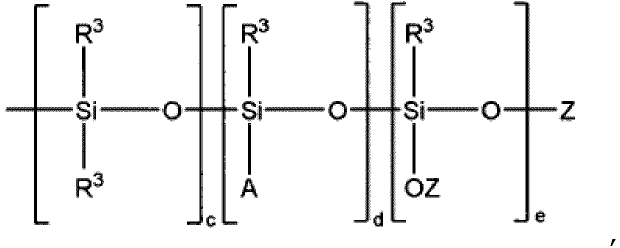 R³ indépendamment les uns des autres signifiant respectivement un radical hydrocarboné monovalent saturé ou insaturé, éventuellement substitué par les éléments N, P, S, O, Si et halogène, comportant 1 à 200 atome(s) de carbone par radical,
A signifiant un radical de formule R⁶-[NR⁷-R⁸-]_{f}NR⁷2,
R⁶ signifiant un radical hydrocarboné divalent linéaire ou ramifié comportant 3 à 18 atomes de carbone,
R⁷ signifiant un atome d'hydrogène, un radical alkyle comportant 1 à 8 atome(s) de carbone ou un radical acyle,
R⁸ signifiant un radical hydrocarboné divalent comportant 1 à 6 atome(s) de carbone,
b étant un nombre de 1 à 2000,
c étant 0 ou un nombre de 1 à 2000,
d étant un nombre de 1 à 1000,
e étant 0 ou un nombre de 1 à 5,
f étant 0, 1, 2, 3 ou 4,
Z signifiant hydrogène, un radical alkyle comportant 1 à 8 atome(s) de carbone ou 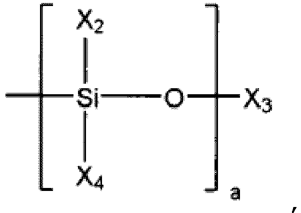 R⁴ signifiant un radical hydrocarboné monovalent comportant 1 à 18 atome(s) de carbone contenant éventuellement des atomes de N et/ou des atomes de O et
R⁵ signifiant un radical hydrocarboné divalent comportant 3 à 12 atomes de carbone contenant éventuellement des atomes de N et/ou des atomes de O, et
b) 0,0001 à 30% en poids de méthionylméthionine.

2. Agent cosmétique selon l'une quelconque des revendications 1, **caractérisé en ce que** fsiloxane est un siloxane à fonctionnalité amine, de préférence un copolymère d'unités 3-(2-aminoéthylamino)propylméthylsiloxyle et diméthylsiloxyle.

3. Agent cosmétique selon la revendication 1 ou 2, **caractérisé en ce que** X₄ est choisi dans le groupe constitué par aminométhyle-, méthylaminométhyle-, diméthylaminométhyle-, diéthylaminométhyle-, dibutylaminométhyle-, cyclohexylaminométhyle-, morpholinométhyle-, pipéridinométhyle-, pipérazinométhyle-, ((diéthoxyméthylsilyl)méthyl)cyclohexylaminométhyl e-, ((triéthoxysilyl)méthyl)cyclohexylaminométhyle-, anilinométhyle-, 3-diméthylaminopropyl-aminométhyle-, bis(3-diméthylaminopropyl)aminométhyle- et des mélanges de ceux-ci.

4. Agent cosmétique selon l'une quelconque des revendications 1 à 3, **caractérisé en ce que** l'agent contient des polyorganosiloxanes, qui contiennent un radical morpholinométhyle en tant que radical X₄.

5. Agent cosmétique selon l'une quelconque des revendications 1 à 4, **caractérisé en ce que** l'agent - par rapport à son poids - contient en outre du tridécanol éthoxylé ramifié (dénomination INCI : Trideceth-5) ou du α-iso-tridécyl-ω-hydroxypolyglycoléther (dénomination INCI : Trideceth-10) ou leurs mélanges.

6. Agent cosmétique selon l'une quelconque des revendications 1 à 5, **caractérisé en ce que** l'agent la méthionylméthionine est un mélange de L-méthionyl-L-méthionine, de L-méthionyl-D-méthionine, D-méthionyl-L-méthionine et de D-méthionyl-D-méthionine.

7. Agent de traitement capillaire selon l'une quelconque des revendications 1 à 5, **caractérisé en ce que** la méthionylméthionine est la L-méthionyl-L-méthionine et/ou la D-méthionyl-D-méthionine.

8. Agent cosmétique selon l'une quelconque des revendications 1 à 7, contenant - à chaque fois par rapport à son poids -
a) 0,0001 à 20% en poids d'un polyorganosiloxane, qui est connu sous la dénomination INCI Amodimethicone/Morpholinomethyl Silsesquioxane Copolymer, et
b) 0,0001 à 30% en poids de méthionylméthionine.

9. Agent cosmétique selon l'une quelconque des revendications 1 à 7, contenant - à chaque fois par rapport à son poids -
a) 0,0001 à 20% en poids d'un polyorganosiloxane, qui peut être obtenu par la transformation d'un siloxane contenant des groupes silanol ou bien des groupes alcoxyle et silanol, avec un trialcoxysilane ou bien un mélange de dialcoxysilanes et trialcoxysilanes, les dialcoxysilanes et trialcoxysilanes présentant un radical morpholinyle, et
b) 0,0001 à 30% en poids de méthionylméthionine.

10. Utilisation de méthionylméthionine dans un agent cosmétique, de préférence dans un agent de traitement capillaire, comprenant un polyorganosiloxane de formule (I) 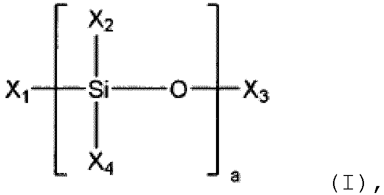 dans laquelle
X₁ et X₂ indépendamment l'un de l'autre signifient OH, OR¹, R², O-PDMS ou O-fsiloxane,
X₃ signifie hydrogène ou un radical hydrocarboné monovalent comportant 1 à 8 atome(s) de carbone par radical, PDMS ou fsiloxane,
X₄ est un radical de formule
-CH₂NHR⁴, -CH₂NR⁴₂ ou 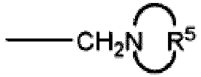 et
a est un nombre de 1 à 100,
R¹ signifiant un radical alkyle comportant 1 à 8 atome(s) de carbone,
R² signifiant un radical hydrocarboné monovalent saturé ou insaturé, éventuellement substitué par les éléments N, P, S, O, Si et halogène, comportant 1 à 200 atome (s) de carbone par radical,
PDMS représentant 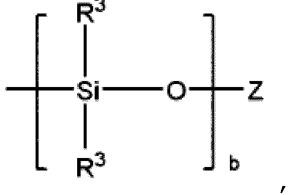 fsiloxane représentant 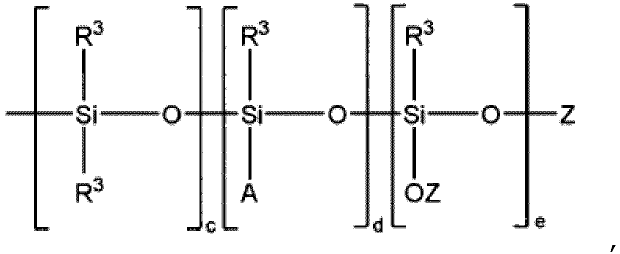 R³ indépendamment les uns des autres signifiant respectivement un radical hydrocarboné monovalent saturé ou insaturé, éventuellement substitué par les éléments N, P, S, O, Si et halogène, comportant 1 à 200 atome (s) de carbone par radical,
A signifiant un radical de formule R⁶-[NR⁷-R⁸-]_{f}NR⁷2, R⁶ signifiant un radical hydrocarboné divalent linéaire ou ramifié comportant 3 à 18 atomes de carbone,
R⁷ signifiant un atome d'hydrogène, un radical alkyle comportant 1 à 8 atome(s) de carbone ou un radical acyle,
R⁸ signifiant un radical hydrocarboné divalent comportant 1 à 6 atome(s) de carbone,
b étant un nombre de 1 à 2000,
c étant 0 ou un nombre de 1 à 2000,
d étant un nombre de 1 à 1000,
e étant 0 ou un nombre de 1 à 5,
f étant 0, 1, 2, 3 ou 4,
Z signifiant hydrogène, un radical alkyle comportant 1 à 8 atome(s) de carbone ou 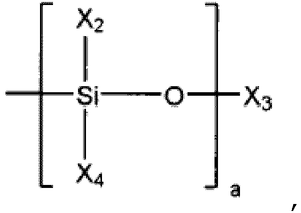 R⁴ signifiant un radical hydrocarboné monovalent comportant 1 à 18 atome(s) de carbone contenant éventuellement des atomes de N et/ou des atomes de O et
R⁵ signifiant un radical hydrocarboné divalent comportant 3 à 12 atomes de carbone contenant éventuellement des atomes de N et/ou des atomes de O,
pour l'amélioration de la résistance à la traction de fibres kératiniques, en particulier de cheveux humains.
